# EUROPEAN PATENT SPECIFICATION

(11) **EP 3 096 704 B1**
(45) Date of publication and mention of the grant of the patent: **10.03.2021**
(21) Application number: 15700759.2
(22) Date of filing: 22.01.2015
(51) Int. Cl.: A61B 46/10, A61B 34/30

(54) **STERILE DRAPE AND ADAPTER FOR COVERING A ROBOTIC SURGICAL ARM AND PREVENTING CONTAMINATION OF A STERILE FIELD**
STERILES TUCH UND ADAPTER ZUM ABDECKEN EINES ROBOTISCHEN CHIRURGISCHEN ARMS UND ZUR VERHINDERUNG DER KONTAMINATION EINES STERILEN FELDES
DRAP STÉRILE ET ADAPTATEUR POUR RECOUVRIR UN BRAS CHIRURGICAL ROBOTIQUE ET EMPÊCHER LA CONTAMINATION DE CHAMP STÉRILE

(30) Priority: 22.01.2014 US 201461930425 P; 24.02.2014 US 201461944037 P
(43) Date of publication of application: 30.11.2016
(73) Proprietor: KB Medical SA, 1024 Ecublens (CH)
(72) Inventor: NUSSBAUMER, Billy, 2017 Boudry (CH); KOSTRZEWSKI, Szymon, CH-1004 Lausanne (CH)
(74) Representative: Graham Watt & Co LLP
(86) International application number: PCT/EP2015/051273
(87) International publication number: WO 2015/110542

(56) References cited:
- WO-A1-98/02107
- WO-A1-2013/160239
- US-A- 4 799 779
- US-A1- 2006 161 138
- US-A1- 2010 319 713

## Description

### Background

Robotic-assisted surgical systems have been developed to improve surgical precision and enable the implementation of new surgical procedures. For example, robotic systems have been developed to sense a surgeon's hand movements and translate them to scaled-down micro-movements and filter out unintentional tremors for precise microsurgical techniques in organ transplants, reconstructions, and minimally invasive surgeries. Other robotic systems are directed to telemanipulation of surgical tools such that the surgeon does not have to be present in the operating room, thereby facilitating remote surgery. Feedback-controlled robotic systems have also been developed to provide smoother manipulation of a surgical tool during a procedure than could be achieved by an unaided surgeon.

However, widespread acceptance of robotic systems by surgeons and hospitals is limited for a variety of reasons. Current systems are expensive to own and maintain. They often require extensive preoperative surgical planning prior to use, and they extend the required preparation time in the operating room. They are physically intrusive, possibly obscuring portions of a surgeons field of view and blocking certain areas around the operating table, such that a surgeon and/or surgical assistants are relegated to one side of the operating table. Current systems may also be non-intuitive or otherwise cumbersome to use, particularly for surgeons who have developed a special skill or "feel" for performing certain maneuvers during surgery and who find that such skill cannot be implemented using the robotic system. Finally, robotic surgical systems may be vulnerable to

malfunction or operator error, despite safety interlocks and power backups. WO 2013/160239 A1 discloses a sterile drape for covering a surgical robot arm.

US 4799779 A discloses a surgical drape for covering a microscope having side sleeves.

Spinal surgeries often require precision drilling and placement of screws or other implements in relation to the spine, and there may be constrained access to the vertebrae during surgery that makes such maneuvers difficult. Catastrophic damage or death may result from improper drilling or maneuvering of the body during spinal surgery, due to the proximity of the spinal cord and arteries. Common spinal surgical procedures include a discectomy for removal of all or part of a disk, a foraminotomy for widening of the opening where nerve roots leave the spinal column, a laminectomy for removal of the lamina or bone spurs in the back, and spinal fusion for fusing of two vertebrae or vertebral segments together to eliminate pain caused by movement of the vertebrae.

Spinal surgeries that involve screw placement require preparation of holes in bone (e.g., vertebral segments) prior to placement of the screws. Where such procedures are performed manually, in some implementations, a surgeon judges a drill trajectory for subsequent screw placement on the basis of pre-operative CT scans. Other manual methods which do not involve usage of the pre-operative CT scans, such as fluoroscopy, 3D fluoroscopy or natural landmark-based, may be used to determine the trajectory for preparing holes in bone prior to placement of the screws. In some implementations, the surgeon holds the drill in his hand while drilling, and fluoroscopic images are obtained to verify if the trajectory is correct. Some surgical techniques involve usage of different tools, such as a pedicle finder or K-wires. Such procedures rely strongly on the expertise of the surgeon, and there is significant variation in success rate among different surgeons. Screw misplacement is a common problem in such surgical procedures.

Image-guided spinal surgeries involve optical tracking to aid in screw placement. However, such procedures are currently performed manually, and surgical tools can be inaccurately positioned despite virtual tracking. A surgeon is required to coordinate his real-world, manual manipulation of surgical tools using images displayed on a two dimensional screen. Such procedures can be non-intuitive and require training, since the surgeon's eye must constantly scan both the surgical site and the screen to confirm alignment. Furthermore, procedural error can result in registration inaccuracy of the image-guiding system, rendering it useless, or even misleading.

Certain force feedback systems are used by surgeons in certain procedures; however such systems have a large footprint and take up valuable, limited space in the operating room. These systems also require the use of surgical tools that are specially adapted for use with the force feedback system, and the training required by surgeons to operate such systems can be significant. Moreover, surgeons may not be able to use expertise they have developed in performing spinal surgeries when adapting to use of the current force feedback systems. Such systems, while precise, may require more surgical time and more operating room preparation time to ready placement of the equipment for surgery. Thus, there is a need for systems and apparatus that provide enhanced precision in performing surgeries such as spinal surgeries.

### Summary

Described herein are a sterile drape and adapter for use with a robotic surgical system, for example, during spinal surgery. The invention is defined by claim 1. Further embodiments of the invention are defined by the dependent claims.

In certain embodiments, the sterile drape and adapter cover a robotic surgical arm and prevent contamination of a sterile field. The sterile drape may attached to the robotic arm via a sterile adapter that ensures the drape is tightly stretched over the tool holder and robot interface to provide a structure that provides for repeatable and rigid positioning of the sterile drape. Tightly stretching the drape over the sterile adapter and tightening the tool holder to the robotic arm using, for example, a tightening screw, reduces the likelihood of folds in the drape between the told holder and robot interface. Thus, errors caused by inaccurate positioning of the tool holder relative to the robot interface are minimized. Moreover, tightly stretching the drape over sterile adapter ensures that the sterile adapter is easy to perforate when the sterile tool holder is installed. As the tightening screw securely is tightened to the appropriate torque specification, a hermetically sealed zone is formed by the tool holder, sterile adapter, and robotic arm. The hermetically sealed zone contains the perforated sections of the sterile drape, thus ensuring the robotic system may be used in a sterile environment even after the tool support is attached to the robotic arm.

In certain embodiments, the disclosed technology includes a sterile drape for covering a surgical robotic arm and preventing contamination of a sterile field. In some implementations, the sterile drape includes a flexible covering with a sterile outer surface. The covering may be at least partially conformable to a surgical robotic arm. The sterile drape may include an embedded connector configured (e.g., positioned on the flexible covering and sized) to permit electrical contact between the surgical robotic arm (e.g., an actuator of the robotic arm) and a sterile manipulator (e.g., sterile handle) of the robotic arm when the sterile manipulator is separated from the surgical robotic arm by the flexible covering. In some implementations, the sterile drape is disposable (e.g., a single-use product).

In certain embodiments, the disclosed technology includes a rigid or semi-rigid sterile adapter (e.g., made from a hard plastic, a polymer, or a composite material) for securing a sterile drape over a surgical robotic arm to prevent contamination of a sterile field. In some implementations, the sterile adapter includes a rigid or semi-rigid collar (e.g., ring) configured to mount (e.g., snap-mount) onto an interface of the surgical robotic arm. The sterile adapter may include a rigid or semi-rigid body extending from the collar and shaped to conform to a portion of the surgical robotic arm to tightly secure a flexible drape in place (e.g., with no folds) over the portion of the surgical robotic arm when the drape is attached to the adapter. In some implementations, the sterile adapter is disposable (e.g. a single-use product).

In certain embodiments, the disclosed technology includes a sterile drape that is attached (e.g., glued or welded) to a sterile adapter. In some implementations, the sterile adapter that ensures the drape is tightly stretched over the tool holder and robot interface to protect the robotic arm and mobile cart from contaminating the sterile field, and provides a structure that provides for repeatable and rigid positioning of the sterile drape.

The disclosed technology, in certain embodiments, includes a sterile drape for covering a surgical robotic arm and preventing contamination of a sterile field. The sterile drape includes, in certain embodiments, a flexible covering with a sterile outer surface and a sterile drape sleeve configured to receive an electrical plug therethrough, said covering being at least partially conformable to a surgical robotic arm. In certain embodiments, the sterile drape sleeve is configured to be sealed around a portion of the electrical plug using a sterile adhesive tape. The sterile drape sleeve, in certain embodiments, is configured to surround at least a portion of a connector of the surgical robotic arm and extend outwardly from the connector.

In certain embodiments, the connector is configured to permit electrical contact between the surgical robotic arm (e.g., an actuator of the robotic arm) and a sterile manipulator (e.g., sterile handle) of the robotic arm when the sterile manipulator is separated from the surgical robotic arm by the flexible covering.

In certain embodiments, the sterile drape is configured to be attached to the sterile drape before draping the robotic surgical arm. In certain embodiments, the connector is configured to receive the electrical plug after the electrical plug is passed through the sterile drape sleeve. In certain embodiments, the connector is configured to receive the electrical plug after (i) the electrical plug is passed through the sterile drape sleeve and (ii) the sterile drape sleeve is sealed around a portion of the electrical plug using a sterile adhesive tape.

In certain embodiments, the sterile drape is configured to be attached to the sterile drape after draping the robotic surgical arm. In certain embodiments, the sterile drape comprises a drape stiffener configured to assist in passing the electrical plug through the sterile drape sleeve. In certain embodiments, the drape stiffener is made of a semi-rigid material (e.g., rubber, adhesive tape, tape reinforcement). In certain embodiments, the drape stiffener is made of a rigid material (e.g., plastic). In certain embodiments, the drape stiffener is integrated in the sterile drape. In certain embodiments, the drape stiffener is removable. In certain embodiments, the drape stiffener is configured to be inserted before the sterile drape is applied to the robot surgical arm. In certain embodiments, the drape stiffener is configured to be inserted after the sterile drape is applied to the robot surgical arm. In certain embodiments, the drape stiffener is configured to be removed after the sterile drape is applied to the robot surgical arm. In certain embodiments, the drape stiffener is configured to be removed after (i) the sterile drape is applied to the robot surgical arm and (ii) the sterile drape sleeve is sealed around a portion of the electrical plug using a sterile adhesive tape.

In certain embodiments, the disclosed technology includes a sterile drape for covering a surgical robotic arm and preventing contamination of a sterile field. In certain embodiments, the sterile drape includes a flexible covering with a sterile outer surface, said covering being at least partially conformable to a surgical robotic arm. In certain embodiments, the flexible covering includes a drape opening that is configured to be contained within a sealed chamber formed by the surgical robotic arm, a sterile adapter, and a surgical tool holder such that a fastening system may pass through the chamber and the drape opening when the surgical instrument holder is coupled to the surgical robotic arm.

In certain embodiments, the fastening system comprises one or more positioning elements that extend from the surgical robotic arm and engage one or more surgical instrument holder positioning members. In certain embodiments, the one or more positioning elements are one or more pegs. In certain embodiments, the one or more pegs are configured to extend from the robotic arm and engage one or more holes in the surgical instrument holder. In certain embodiments, the fastening system comprises a fastening member configured to securely attach the surgical instrument holder to the surgical robotic arm. In certain embodiments, the fastening member is a member selected from the group consisting of a bolt, nut, screw, and one or more electro magnets.

In certain embodiments, the disclosed technology includes a sterile drape for covering a surgical robotic arm and preventing contamination of a sterile field. In certain embodiments, the sterile drape includes a flexible covering with a sterile outer surface, said covering being at least partially conformable to a surgical robotic arm; and an embedded connector configured (e.g., positioned on the flexible covering and sized) to permit electrical contact between the surgical robotic arm (e.g., an actuator of the robotic arm) and a sterile manipulator (e.g., sterile handle) of the robotic arm when the sterile manipulator is separated from the surgical robotic arm by the flexible covering.

In certain embodiments, the disclosed technology includes a rigid or semi-rigid sterile adapter (e.g., made from a hard plastic, a polymer, or a composite material) for securing a sterile drape over a surgical robotic arm to prevent contamination of a sterile field. In certain embodiments, the sterile adapter includes a rigid or semi-rigid collar (e.g., ring) configured to mount (e.g., snap-mount) onto an interface of the surgical robotic arm; and a rigid or semi-rigid body extending from the collar and shaped to conform to a portion of the surgical robotic arm to tightly secure a flexible drape in place (e.g., with no folds) over the portion of the surgical robotic arm when the drape is attached to the adapter.

In certain embodiments, the sterile drape is disposable (e.g., a single-use product). In certain embodiments, the sterile adapter is disposable (e.g. a single-use product).

In certain embodiments, the disclosed technology includes an apparatus that includes a sterile drape and a sterile adapter. In some implementations, the apparatus is disposable (e.g., a single-use product).

In certain embodiments, the disclosed technology includes a robotic surgical system for performing surgery. In some implementations, the system includes a robotic arm with an end effector, a surgical instrument holder configured to securely hold the surgical instrument, and one or more positioning elements configured to provide accurate and repeatable positioning of the surgical instrument holder in reference to the robotic arm; a manipulator configured to allow robotically-assisted or unassisted positioning and/or movement of the surgical instrument by a user with at least four degrees of freedom to align an axis defined by the surgical instrument at a desired trajectory in relation to a patient situation; a sterile drape configured to protect the robotic arm from contaminating a sterile field.

In certain embodiments, the sterile drape includes a drape connector configured to electrically couple, through the sterile drape, the manipulator to an electrical system of the robotic surgical system covered by the sterile drape, and the one or more positioning elements pass through the sterile drape. In certain embodiments, a sterile adapter is configured to attach to the robotic arm via a robotic interface and tightly stretch the sterile drape to assure repeatable and rigid positioning of the sterile drape. In certain embodiments, the surgical instrument holder is attached to the robotic arm using a fastening system configured to pass through the sterile adapter and the sterile drape.

In certain embodiments, the surgical instrument comprises a surgical instrument guide configured to hold and/or restrict movement of a second surgical instrument there through. In certain embodiments, the second surgical instrument is a member selected from the group consisting of: a drill bit, tap, screw driver, and awl. In certain embodiments, the second surgical instrument is a drill bit and the surgical instrument guide is a drill guide. In certain embodiments, the robotic surgical system is for use in spinal surgery.

In certain embodiments, the one or more positioning elements extend from the robotic arm and engage one or more surgical instrument holder positioning members. In certain embodiments, the one or more positioning elements are one or more pegs. In certain embodiments, the one or more pegs are configured to extend from the robotic arm and engage one or more holes in the surgical instrument holder. In certain embodiments, the one or more positioning elements are configured to pass through the sterile drape.

In certain embodiments, the system includes a mobile cart configured to transport the robotic surgical system, wherein the sterile drape is configured to protect the mobile cart from contaminating a sterile field. In certain embodiments, the sterile drape comprises a first sterile drape to protect the robotic arm and a second sterile drape to protect the mobile cart.
In certain embodiments, the sterile drape comprises printed marks configured to assist in proper draping procedure. In certain embodiments, the system includes one or more holding stripes configured to hold the sterile drape (e.g., securely in place). In certain embodiments, the one or more holding stripes secure a portion of the sterile drape to the robotic arm.

In certain embodiments, the system includes a suppression system configured to remove air from under the sterile drape. In certain embodiments, the suppression system includes at least one member selected from the group consisting of a ventilator and a suction device that pumps out the air from under the sterile drape. In certain embodiments, the sterile handle is coupled to a handle connector via a cable and the handle connector is configured to electrically connect to the drape connector.

In certain embodiments, the electrical system of the robotic surgical system is coupled to a robot connector and the robot connector is configured to electrically connect to the drape connector. In certain embodiments, the robot connector electrically couples the drape connector to the robot connector.

In certain embodiments, the sterile adapter comprises one or more tabs configured to attach to the robotic arm. In certain embodiments, the one or more tabs click onto an interface on the robotic arm. In certain embodiments, the one or more tabs comprise four tabs.

In certain embodiments, the sterile drape is glued or welded to the sterile adapter.

In certain embodiments, the sterile drape passes between the surgical instrument tool holder and the robotic interface. In certain embodiments, the sterile drape is tightly stretched between the surgical instrument tool holder and the robotic interface. In certain embodiments, the surgical instrument holder is a non-conductive material.

In certain embodiments, the disclosed technology includes an exemplary not claimed method of performing surgery with a robotic surgical system. In certain embodiments, the method includes attaching a sterile adapter to a robotic arm of a robotic surgical system via a robotic interface, wherein: a sterile drape is coupled to the sterile adapter and is configured to protect the robotic arm from contaminating a sterile field, and the sterile adapter is configured to tightly stretch the sterile drape to assure repeatable and rigid positioning of the sterile drape; covering a portion of the robotic surgical system with the sterile drape; connecting a drape connector to a handle connector and a robot connector, wherein the sterile drape comprises the drape connector and the drape connector is configured to electrically couple the handle connector to the robot connector; securing a portion of the sterile drape to the robotic arm via one or more holding stripes; attaching the surgical tool holder to the robotic arm using a fastening system configured to pass through the sterile adapter and the sterile drape, wherein: the surgical tool holder is configured to slide along one or more positioning elements, the one or more positioning elements of a robotic arm of the robotic surgical system are configured to cause one or more holes in the sterile drape, and the one or more position elements are configured to provide accurate and repeatable positioning of the surgical instrument holder in reference to the robotic arm; moving a mobile cart transporting the robotic surgical system comprising the robotic arm in proximity to an operating table, wherein the robotic arm has an end effector comprising a surgical instrument holder holding a surgical instrument attached thereto; stabilizing the mobile cart; maneuvering the robotic arm to a desired position to align an axis defined by the surgical instrument at a desired trajectory in relation to a patient situation; and fixing the position of the robotic arm (and, therefore, the position of the surgical instrument).

In certain embodiments, the exemplary method includes maneuvering the second surgical instrument through the surgical instrument guide. In certain embodiments, the method includes maneuvering a drill bit through a drill bit guide. In certain embodiments, the sterile drape is configured to cover the robotic arm and mobile cart and protect the robotic arm and mobile cart from contaminating a sterile field. In certain embodiments, the fastening system goes through the sterile drape. In certain embodiments, the one or more holding stripes wrap around the robotic arm. In certain embodiments, a suppression system configured to remove air from under the sterile drape. In certain embodiments, the suppression system comprises at least one member selected from the group consisting of a ventilator and a suction device that pumps out the air from under the sterile drape. In certain embodiments, stabilizing the mobile cart includes extracting one or more rigid legs on the mobile cart such that the mobile cart rests on the one or more rigid legs of the mobile cart. In certain embodiments, stabilizing the mobile cart comprises retracting one or more wheels on the mobile cart such that the mobile cart rests on one or more rigid legs of the mobile cart.

In certain embodiments, the method includes, prior to maneuvering the robotic arm to a desired position, obtaining or accessing a CT scan, 3D CT scan, fluoroscopy, 3D fluoroscopy, or natural landmark-based image of the patient situation. In certain embodiments, the desired trajectory is a desired path of the surgical tool.

### Brief Description of the Figures

The foregoing and other objects, aspects, features, and advantages of the present disclosure will become more apparent and better understood by referring to the following description taken in conjunction with the accompanying drawings, in which:
FIG. 1 is an illustration of an example robotic surgical system;
FIG. 2 is an illustration of an example sterile adapter for use with a robotic surgical system;
FIG. 3 is an illustration of an example sterile drape coupled, via glue or welding, to a sterile adapter;
FIGS. 4A-B are illustrations of an example sterile adapter applied to a robotic surgical system;
FIG. 5 is an illustration of an example sterilization system with a sterile adapter and sterile drape attached to a robotic arm;
FIG. 6 is an illustration of an example a sterilization system with a sterile adapter and sterile drape attached to a robotic arm;
FIGS. 7A-C are illustrations of an example sterilization system applied to a robotic surgical system;
FIG. 8 is a flowchart of an example method of performing surgery with a robotic surgical system;

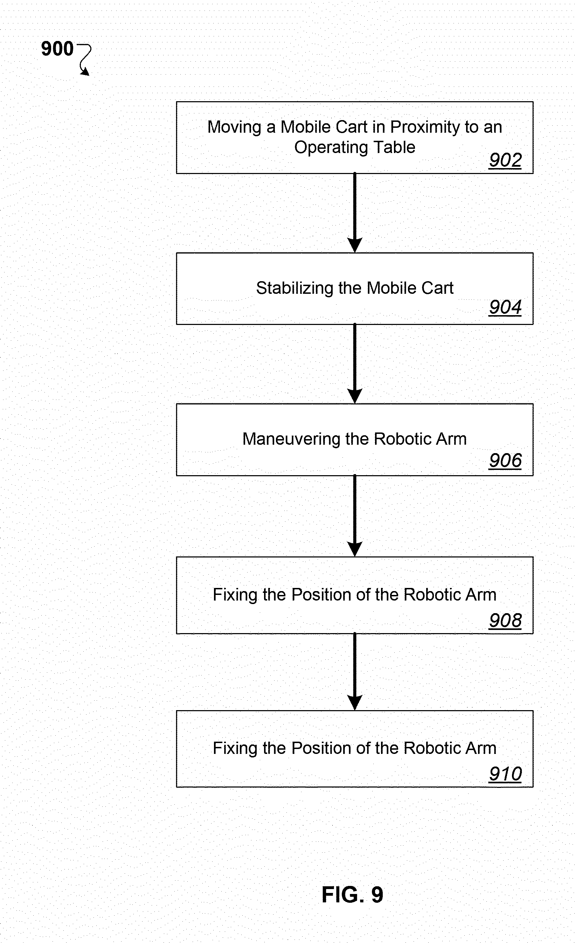

FIG. 9 is a flowchart of an example of a method for performing a minimally invasive surgery using a robotic surgical system as a drill guide;
FIG. 10 is an illustrations of an example system for passing electrical signals through a sterile drape;
FIG. 11A-D are illustrations of the processor applying a sterile drape that allows passage of electrical signals through the drape;
FIGS. 12A-D are illustrations of an example stiffeners for improving the ability to pass an electrical plug through a sleeve of a sterile drape;
FIG. 13 is an illustrations of an example system for passing electrical signals through a sterile drape; and
FIG. 14 is an illustration of an example sterile drape for use with a robotic surgical system.

The features and advantages of the present disclosure will become more apparent from the detailed description set forth below when taken in conjunction with the drawings, in which like reference characters identify corresponding elements throughout. In the drawings, like reference numbers generally indicate identical, functionally similar, and/or structurally similar elements.

### Detailed Description

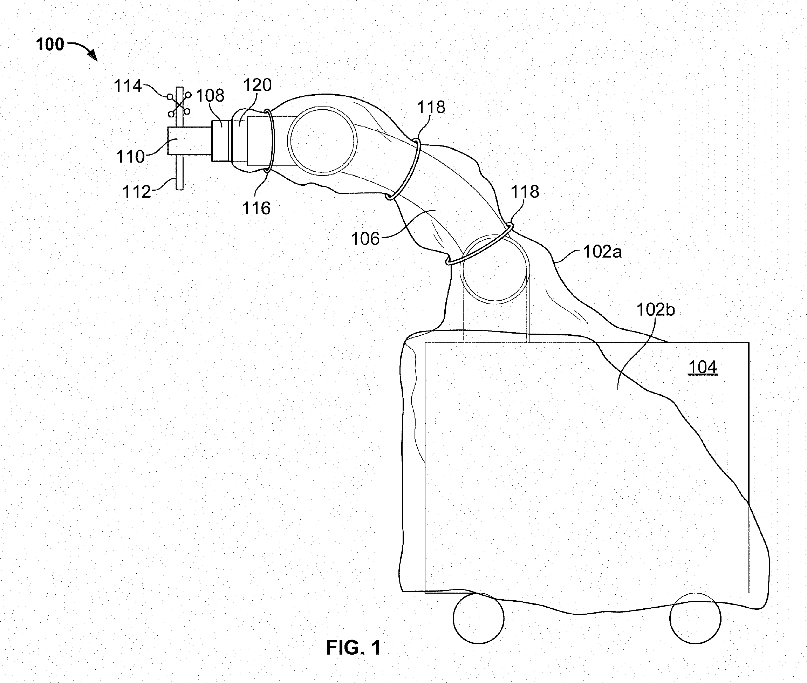

FIG. 1 illustrates an example robotic surgical system 100. In some implementations, one or more surgeons, surgical assistants, surgical technologists and/or other technicians, perform an operation on a patient using a robotic-assisted surgical system 100. In the operating room the surgeon may be guided by the robotic system 100 to accurately execute an operation. The robotic surgical system 100 may be transported in and out of an operating room using a mobile cart 104. Accordingly, the robotic surgical system 100, including the mobile cart 104, must be sterilized when used in the operating room. The system may be sterilized by applying a sterile drape 102 to a portion of the system 100, including at least part of the robotic arm 106 and the mobile cart 104. The sterile drape 102 may consist of a single drape or several pieces, such as sterile cover 102a for covering the robotic arm 106 and sterile drape 102b for covering the mobile cart 104.

In some implementations, the sterile drape 102 is attached (e.g., glued or welded) to a sterile adapter 108. The sterile adapter 108 may be attached (e.g., clipped) to an interface on the robotic arm 106. The sterile adapter 108 ensures the drape 102 is tightly stretched over the interface between the tool holder 110 and robot interface to protect the robotic arm 106 and mobile cart 104 from contaminating the sterile field, and provides a structure that provides for repeatable and rigid positioning of the sterile drape 102. Tightly stretching the drape 102 between the robot interface and tool holder 110 and tightening the tool holder to the robotic arm using, for example, a tightening screw, reduces the likelihood of folds in the drape 102 between the told holder 110 and robot interface. Thus, errors between the robot model and the actual situation because of the position of the tool holder 110 relative to the robot interface are minimized. If folds are present, the positioning of the tool holder 110 relative to the robot interface will be different than anticipated and the robotic surgical system 100 may have difficulty positioning tools held by the tool holder 110 appropriately.

A sterile tool holder 110 may be connected to the robotic arm 106 through the sterile drape 102. The tool holder 110 may hold sterile surgical instruments (e.g., tool guide 112) and may be coupled to a navigation marker 114. Thus, the disclosed technology enables a robotic surgical system 100 to be used in a sterile operating room without having to sterilize each individual component of the system. Only the components outside of the sterile drape 102 (e.g., the optical marker 114, surgical instruments (e.g., 112), and tool holder 110 must be sterilized individually.

A band 116 may be provided around the force sensor 120 mounted on the robotic arm. Band 116 may provide force sensor 120 protection. For example, in some implementations, the force sensor measures forces applied to all parts attached to it (e.g., sterile adapter, tool guide, optical marker, etc.). The sterile drape is attached to the sterile adapter and when, for example, the robotic arm moves or somebody touches the drape, the drape can tear on the force sensor and/or impact the measurements by the force sensor. By adding force sensor protection (e.g., band 116), this problem is mitigated. The drape 102 may also be secured around the robotic arm 106 using holding stripes 118. In some implementations, the a suppression system, such as a pump, ventilator, or other suction device may be used to remove air from inside the device. In some implementations, the holding strips 118 are used in combination with a suppression system.

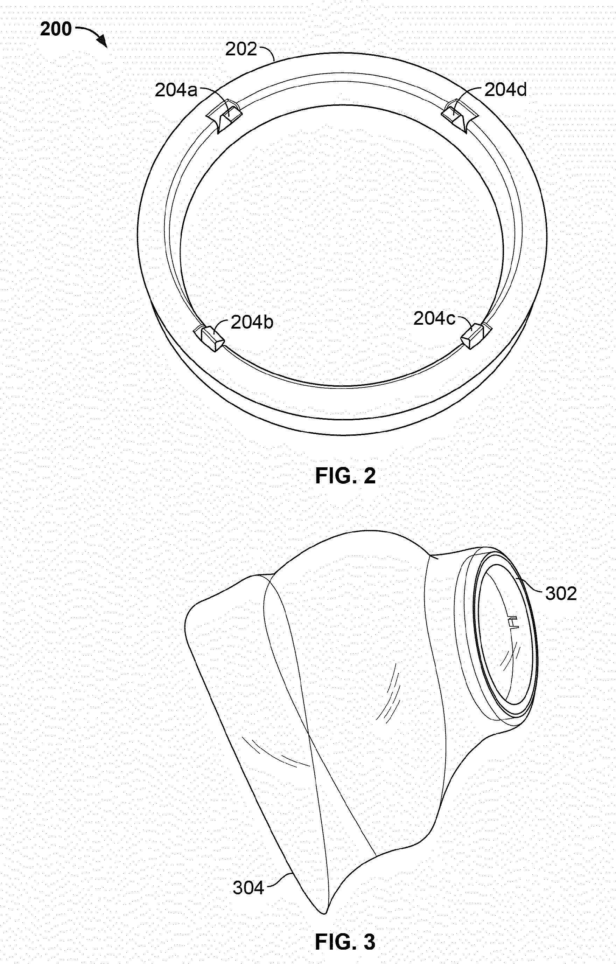

FIG. 2 is an illustration of an example sterile adapter 200 for use with a robotic surgical system. The sterile adapter 200 may be a disposable (e.g. a single-use product). For example, a new sterile adapter 200 may be used for every surgical procedure. In some implementations, the sterile adapter 200 is a rigid or semi-rigid device. It may be made from a hard plastic, polymer, or a composite material. In some implementations, the sterile adapter 200 secures a drape over a surgical robotic arm to prevent contamination of a sterile field.

The sterile adapter 200 may include a rigid or semi-rigid collar 202 (e.g., ring or a hollow cylindrical structure) configured to mount (e.g., snap-mount) onto an interface of the surgical robotic arm. The sterile adapter 200 may include a rigid or semi-rigid body 204 extending from the collar and shaped to conform to a portion of the surgical robotic arm to tightly secure a flexible drape in place (e.g., with no folds) over the portion of the surgical robotic arm when the drape is attached to the adapter 200.

In some implementations, the body 204 is one or more tabs 204a-d (e.g., 3, 4, 5, 6, 7, or 8 tabs) that engage an interface on the robot. The tabs may "click" into the interface to provide easy and secure mounting of the sterile adapter, and hence sterile drape, on the robot. The sterile drape may be glued or welded to the sterile adapter 200 (e.g., during manufacturing). The adapter 200 ensures that the drape is tightly stretched over the tool holder and robot interface to provide repeatable and rigid positioning of the tool holder relative to the robotic arm. FIG. 3 is an illustration of an sterile drape 304 coupled, via glue or welding, to a sterile adapter 302. In some implementations, the sterile drape 304 is glued or welded to the sterile adapter 302. After the welding/gluing dries the part of the drape inside the sterile adapter 302 is stretched. This portion of the drape (i.e., the stretched portion inside of the sterile adapter 302 is the only part of the drape that is located between the tool holder and the robotic arm. As shown in FIG. 3, the sterile drape 304 is tightly stretched over the opening of the sterile adapter 302. When the sterile adapter 302 is attached to the robotic arm (e.g., clicked into the interface on the robotic arm), the end of the robotic arm will be covered by the sterile drape 304 that is stretched over the opening of the sterile adapter 302. As described below, in some implementations, positioning elements and a tightening screw will protrude through the opening of the sterile adapter and piece the sterile drape when the tool support is applied to the robotic arm.

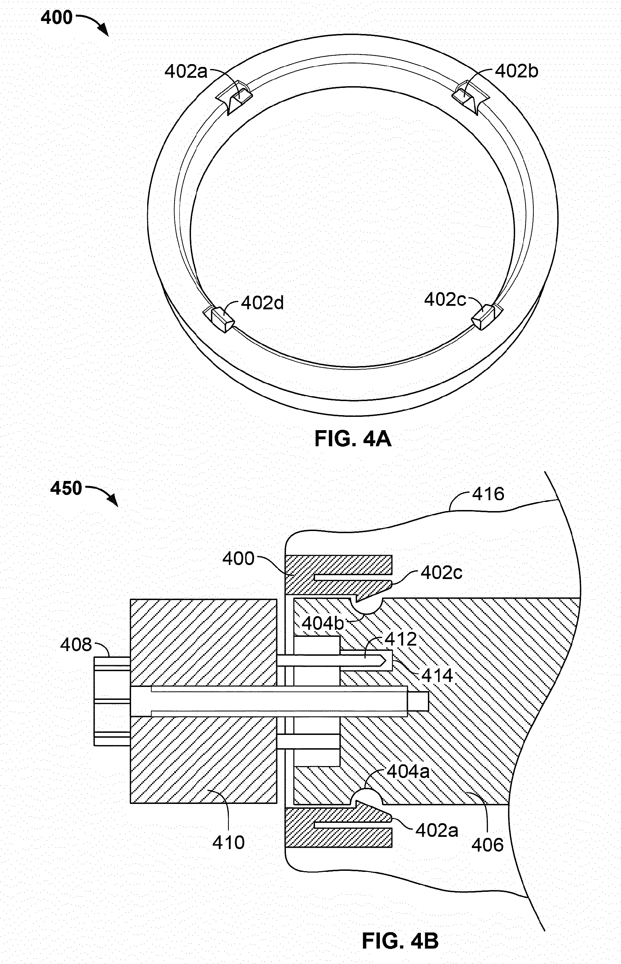

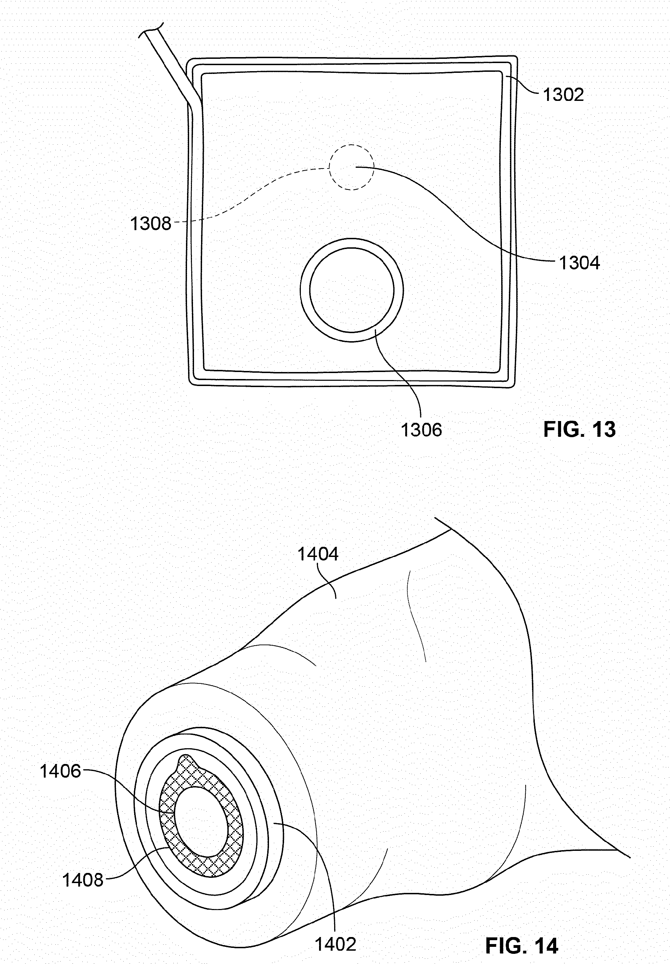

FIG. 4A-B illustrate an example sterile adapter 400 applied to a robotic surgical system 450. Tabs/clips 402 engage grooves 404 on the interface 406 of the robot arm. In some implementations, the tabs/clips 402 include a single tab/clip. The tabs/clips may "click" to engage the groove(s) 402 on the robot interface 406. After the sterile adapter 400 is attached to the robot interface 406, the tool holder 410 may be attached to the robot. The tool holder 410 may include one or more positioning members 412 (e.g., pins or pegs) that engage one or more holes 414 in the robot interface 406 to provide accurate/repeatable positioning of the tool holder in reference to the robot interface. The one or more positioning members 412 may pierce the sterile drape 416 to engage the hole(s) 414. In some implementations, as shown in FIG. 14, the sterile drape 1404 includes a drape opening 1406 that is large enough to allow passage of the positioning pins without puncturing the drape. The sterile adapter 1402 stretches the drape to avoid folds as described above. The drape opening 1406 is configured to be located inside the sterile adapter 1402 (i.e., the sterile drape does is not on the inside of the sterile adapter). A dust cover 1408 may be placed over the drape opening 1406 and attached to the drape 1404. The dust cover 1408 may be configured to attach to the drape 1404 using an adhesive material. The dust cover 1408 may be removed after the drape 1404 is installed, for example, on the robotic surgical arm, thus reducing contamination of the sterile field. After the sterile drape 1404 is installed, the dust cover 1408 may be removed and the tool holder may be installed.

In some implementations, the position member(s) 412 may be uniquely shaped to ensure that the tool holder 410 is always appropriately positioned/ oriented on the robot interface 406. In some implementations, the positioning member(s) is/are located on the robot interface 406 and the hole(s) 414 is/are located on the tool holder 410 (i.e., opposite of what is shown in FIG. 4B).

The tool holder 410 may be securely attached to the robot using a tightening screw 408. The tightening screw 408 may be tightened using a torque wrench or other torque-limited tool. In some implementations, the wrench is disposable and/or destroyed after achieving a certain torque. When the tightening screw is tightened, it pieces the sterile drape 416 and engages threads on the robot interface 406. As discussed above, the sterile drape 416 is tightly stretched over the sterile adapter 400, thus when the tightening screw 408 is tightened to the appropriate torque setting, only a single layer of the sterile adapter 400 material is between the tool holder 410 and the robot interface 406. Moreover, tightly stretching the drape 416 over sterile adapter ensures that the sterile adapter 408 is easy to perforate (e.g., by the position member(s) and the tightening screw) when the sterile tool holder 410 is installed. In some implementations, the a bolt or nut is used to securely attached the tool holder 410 to the robot. In some implementations, electromagnet(s) is/are used to securely attached the tool holder 410 to the robot. In some implementations, the tightening screw 408 and the positioning member(s) 414 protrude through the sterile drape 416. In some implementations, these perforations (e.g., holes in the sterile drape 416) are in the inner space formed by the robot interface 406, the sterile adapter 400, and the tool holder 410. In some implementations, this inner space is hermetically sealed by these three components when the tightening screw 408 is torqued to the appropriate specification.

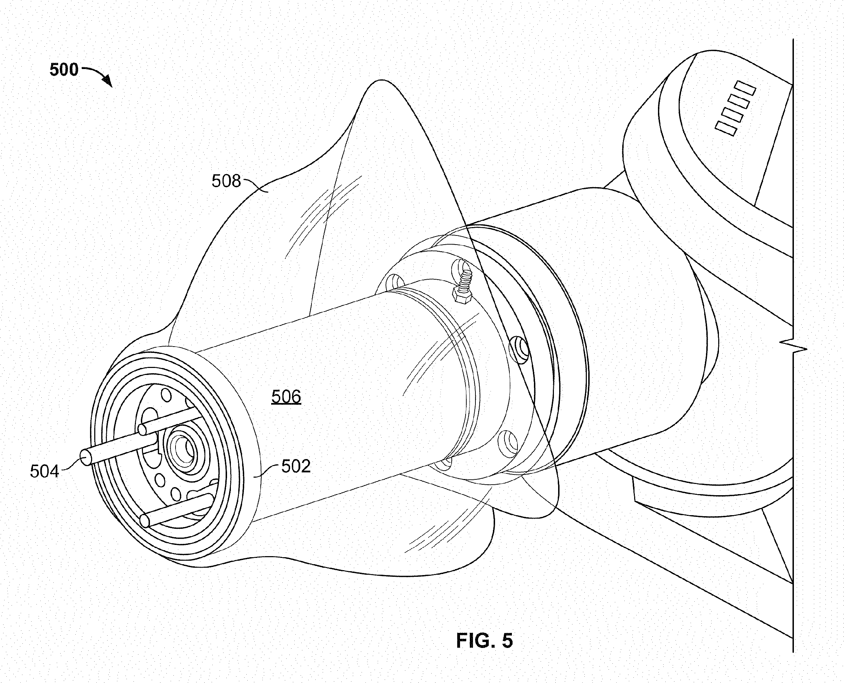

FIG. 5 illustrates an example sterilization system 500 with a sterile adapter 502 and sterile drape 508 attached to a robotic arm 510. In this example, pegs 504(three pegs as shown in FIG. 5) are protruding from the robot interface 506. When the sterile adapter 502 is attached to the robot interface, the pegs 504 protrude through the sterile drape 508. When the tool support is attached, holes in the tool support engage the pegs and ensure the tool support is properly installed and oriented relative to the robotic arm. In some other implementations, the robotic interface 506 includes holes and the tool support includes pegs that engage the holes when the tool support is attached to the robotic interface.

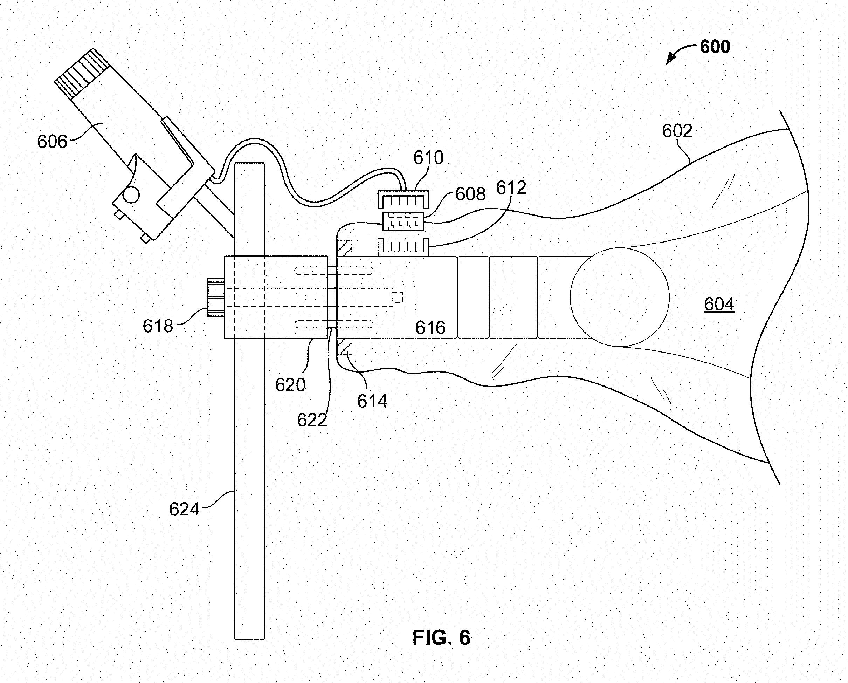

FIG. 6 illustrates an example sterilization system 600 with a sterile adapter 614 and sterile drape 602 attached to a robotic arm 604. In some implementations, the system 600 includes a sterile drape 602 for covering a surgical robotic arm 604 and preventing contamination of a sterile field.

The surgical instrument holder 620 may be attached to the interface 616 via a tightening screw 618. The tightening screw 618 may protrude through the sterile drape 602 that is tightly stretched over the opening of the sterile adapter 614. The robot interface 616 may include one or more positioning elements 622 (e.g., pegs or pins) configured to provide accurate and repeatable positioning of the surgical instrument holder 620 in reference to the robotic arm. In the example shown in FIG. 6, the surgical instrument holder 620 is holding a drill bit guide 624. A user may move the surgical instrument 624 by grasping a handle 606 (e.g., sterile handle). An example handle 606 is discussed in U.S. Patent Application No. 14/597883, the contents of which are hereby incorporated by reference in their entirety.

In certain embodiments, the handle 606 adds functionalities and an interface to existing surgical tools such that the robotic system may be commanded from the sterile field during surgery. The handle 606 permits a user, such as a surgeon, to physically manipulate the location of the end-effector of a robotic surgical system from a sterile field. The handle 606 may include an input device that allows the user to limit the movement of the end-effector, such as limiting the movement to translations or rotations only. The handle 606 may detect the presence of a user's hand. This ensures the end effector is only moved when the user manipulates the handle 606 and reduces the likelihood that the end-effector is moved unintentionally. For example, robotic surgical system may permit the movement of the end-effector only in circumstances when the presence detector is activated (e.g., a hand of a surgeon is detected as present because the surgeon is holding the sterile handle). The handle 606, in certain embodiments, is configured such that it may be used in a sterile environment. The design of the handle 606, in certain embodiments, permits rapid mounting of the handle on a surgical tool. The handle 606 may be designed to avoid tight spaces between various components of the handle 606, thereby simply the sterilization process.

In some implementations, the sterile drape 602 includes a flexible covering with a sterile outer surface. The sterile drape may be partially conformable to a surgical robotic arm. The sterile drape may include an embedded connector 608 configured (e.g., positioned on the flexible covering and sized) to permit electrical contact between the surgical robotic arm 604 (e.g., an actuator of the robotic arm) and a sterile manipulator 606 (e.g., sterile handle) of the robotic arm 604 when the sterile manipulator 606 is separated from the surgical robotic arm 604 by the flexible covering. In some implementations, the sterile drape 602 is disposable (e.g., a single-use product).

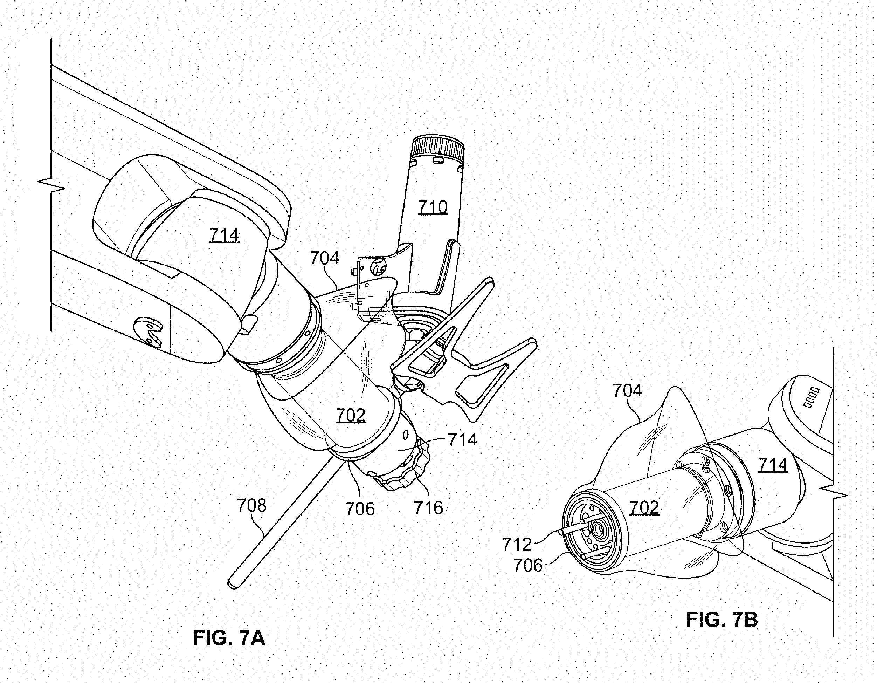

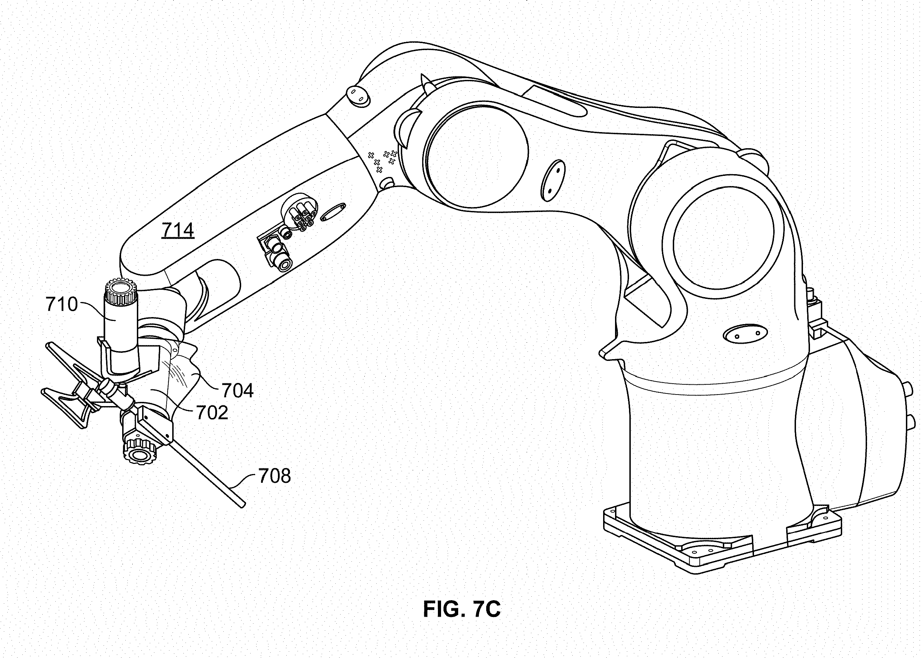

FIGS. 7A-C illustrate an example sterilization system applied to a robotic surgical system. In some implementations, the robotic surgical system is for performing surgery, such as spinal surgery. The robotic surgical system may include a robotic arm 714 with an interface 702 for engaging a sterile adapter 706. The sterile adapter 706 may be configured to attach to the robotic arm 714 via a robotic interface 706 and tightly stretch the sterile drape 704 to assure repeatable and rigid positioning of the sterile drape 704. The sterile drape 704 may be configured to protect the robotic arm from contaminating a sterile field.

In some implementations, the sterile drape includes a drape connector configured to electrically couple, through the sterile drape, the manipulator (e.g., handle 606) to an electrical system of the robotic surgical system covered by the sterile drape. The manipulator 710 (e.g., a handle for grasping by a user) may be coupled to a handle connector via a cable and the handle connector is configured to electrically connect to the drape connector. The electrical system of the robotic surgical system may be coupled to a robot connector and the robot connector may be configured to electrically connect to the drape connector. Thus, the robot connector may electrically couple the drape connector to the robot connector, through the sterile drape 704.

In some implementations, a surgical instrument holder 714 is configured to securely hold the surgical instrument 708. The surgical instrument holder 714 may be attached to the interface 702 via a tightening screw 716. The tightening screw 716 may protrude through the sterile drape 704 that is tightly stretched over the opening of the sterile adapter 706. The robot interface 702 may include one or more positioning elements 712 (e.g., pegs or pins) configured to provide accurate and repeatable positioning of the surgical instrument holder 714 in reference to the robotic arm. The one or more positioning elements 712 may be round or oblong. The surgical instrument holder 714 may include one or more studs or holes that engage the one or more positioning elements 712. The one or more positioning elements 712 may protrude through the sterile drape 704 when the sterile adapter 706 is attached to the interface 702. In some implementations, the one or more positioning elements 712 extend from the robotic arm 714 and engage one or more surgical instrument holder positioning members. For example, the one or more positioning elements 712 may be the one or more pegs are configured to extend from the robotic arm and engage one or more holes in the surgical instrument holder 714.

In some implementations, the robotic surgical system includes a manipulator 710 (e.g., a sterile handle) configured to allow robotically-assisted or unassisted positioning and/or movement of the surgical instrument by a user with at least four degrees of freedom to align an axis defined by the surgical instrument at a desired trajectory in relation to a patient situation.

In some implementations, the surgical instrument is a surgical instrument guide configured to hold and/or restrict movement of a second surgical instrument there through. The second surgical instrument may be a drill bit, tap, screw driver, or awl. For example, in the case of spinal surgery, the second surgical instrument may be a drill bit and the surgical instrument guide may be a drill guide.

In some implementations, the robotic surgical system includes a mobile cart configured to transport the robotic surgical system. The sterile drape may be configured to protect the mobile cart from contaminating a sterile field. In some implementations, the sterile drape includes a first sterile drape to protect the robotic arm 714 and a second sterile drape to protect the mobile cart. In some implementations, the sterile drape may include printed marks configured to assist in proper draping procedure. In some implementations, the drape may be folded in a configuration that makes for easily applying the drape to the robotic system.

In some implementations, the surgical instrument holder is configured to be attached to the robotic arm using a fastening system. The fastening system may be a bolt, nut, screw, or one or more electro magnets.

In some implementations, one or more holding stripes are configured to hold the sterile drape. The one or more holding stripes may secure a portion of the sterile drape to the robotic arm. In some implementations, the system includes a suppression system configured to remove air from under the sterile drape. The suppression system may include a ventilator or a suction device that pumps out the air from under the sterile drape.

In some implementations, the surgical instrument holder 714 is made from a non-conductive material (e.g., plastic). The holder 714 may act as an insulator (prevent electrical conductivity) between the surgical instrument 708 and the robotic arm 714. In some implementations, the surgical instrument holder 714 is conductive, however, a non-conducive pad is placed between the holder 714 and the interface 702.

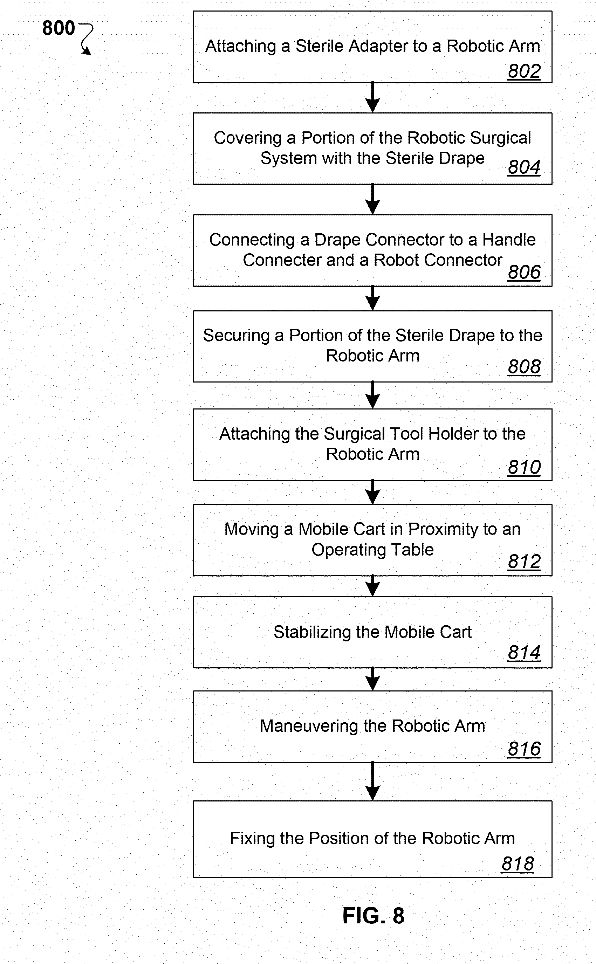

FIG. 8 is a flow chart illustrating an example method 800 of performing a surgical operation with a robotic surgical system. In some implementations, the method 800 includes attaching a sterile adapter to a robotic arm of a robotic surgical system via a robotic interface (802). The a sterile drape may be coupled to the sterile adapter (e.g., glued or welded) and is configured to protect the robotic arm from contaminating a sterile field. The sterile adapter may be configured to tightly stretch the sterile drape to assure repeatable and rigid positioning of the sterile drape.

In some implementations, the method 800 includes covering a portion of the robotic surgical system with the sterile drape (804) and connecting a drape connector to a handle connector and a robot connector (806). The sterile drape may include the drape connector that is configured to electrically couple the handle connector to the robot connector.

In some implementations, the method 800 includes securing a portion of the sterile drape to the robotic arm via one or more holding stripes (808). In some implementations, the method 800 includes attaching the surgical tool holder to the robotic arm using a fastening system configured to pass through the sterile adapter and the sterile drape (810). The surgical tool holder may be configured to slide along one or more positioning elements. The one or more positioning elements of a robotic arm of the robotic surgical system may be configured to cause one or more holes in the sterile drape and also provide accurate and repeatable positioning of the surgical instrument holder in reference to the robotic arm.

In some implementations, the method 800 includes moving a mobile cart transporting the robotic surgical system comprising the robotic arm in proximity to an operating table (812). The robotic arm has an end effector that includes a surgical instrument holder holding a surgical instrument attached thereto.

In some implementations, the method 800 includes stabilizing the mobile cart (814) and maneuvering the robotic arm to a desired position to align an axis defined by the surgical instrument at a desired trajectory in relation to a patient situation (816). In some implementations, the method 800 includes, after maneuvering the robotic arm to the desired position, fixing the position of the robotic arm (and, therefore, the position of the surgical instrument) (818).

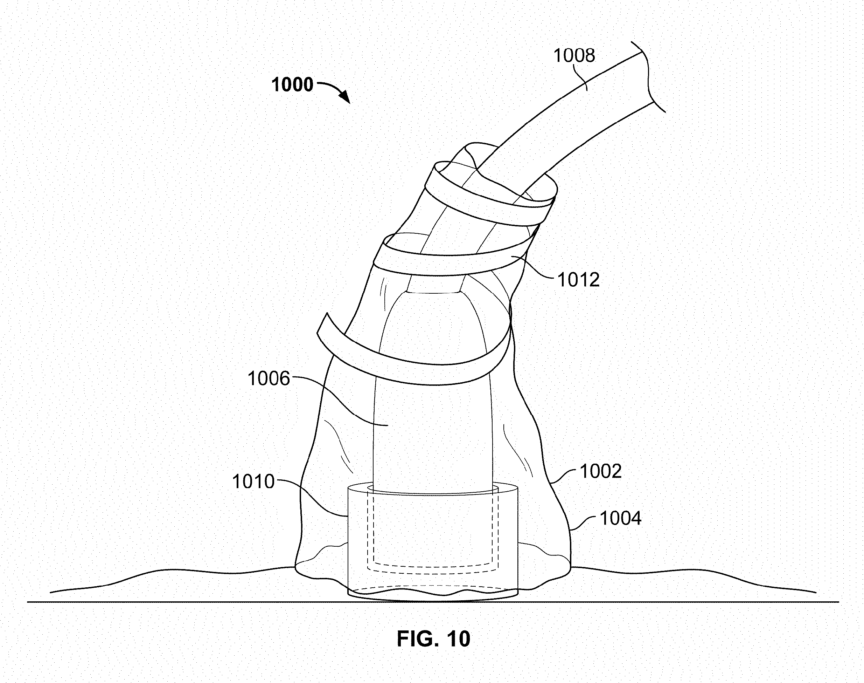

FIG. 10 is an illustration of an example system 1000 for passing electrical signals through a sterile drape. In some implementations, system 1000 is achieved using standard (e.g., disposable) cables and connectors. This requires less investment for the developer and user. Moreover, in situations when each cable may only be used for a signal operation, the cost of the disposable components may be significantly reduced.

The system 100 includes a sterile drape 1002 with a sleeve 1004 which allows for passing a plug 1006 of a cable 1008. The plug 1006 is connected to a sterile handle connector 1010 is used to electrically couple the robotic surgical system to the electrical system of the sterile handle via the cable 1008 and plug 1006. After the plug 1006 is connected to the sterile handle connector 1010, the sleeve 1004 is sealed using sterile tape 1012. The tape 1012 may be wrapped around the sleeve 1004 to seal the sleeve 1004. In some implementations, the sleeve 1004 is part of the sterile drape 1002. In some implementations, the sleeve 1004 is separate from the drape 1002 and the interface between the sleeve 1004 and the sterile drape 1002 is sealed using sterile adhesive tape 1012 or another sterile adhesive material.

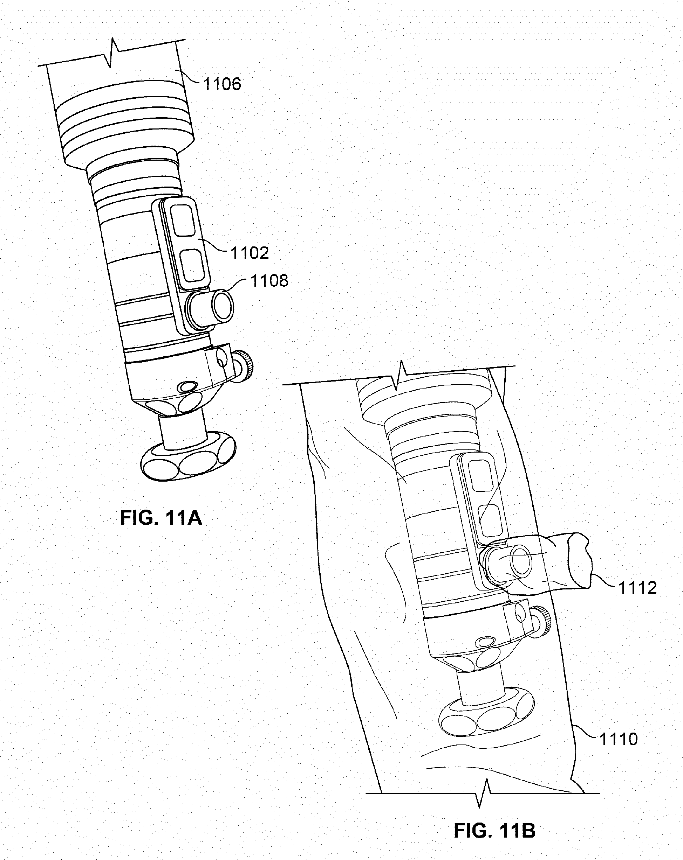

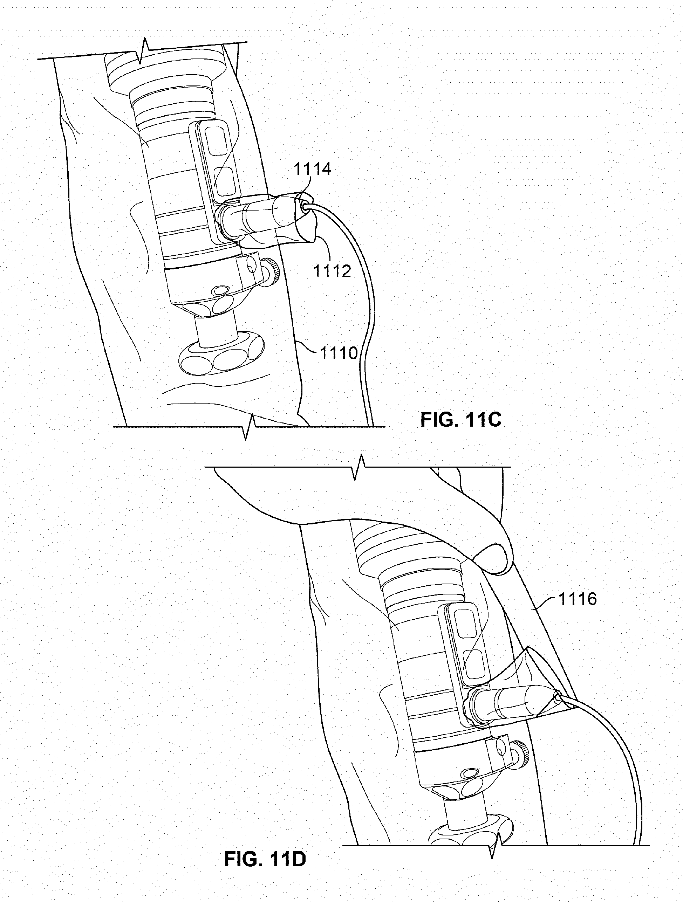

FIGS. 11A-D are illustrations of the processor applying a sterile drape that allows passage of electrical signals through the drape. FIG. 11A illustrates a mode selection panel 1102 on a robotic arm 1106 prior to the installation of a sterile drape. A sterile handle connector 1108 is configured to receive a plug 1114 from a cable connected to the electrical system of the robotic surgical system. FIG. 11B is an illustration of a sterile drape 1110 and sleeve 1112 placed over the connector 1108. After placing the sterile drape 1110 and sleeve 1112 over connector 1108, the plug 1114 is inserted through the hole in the sleeve and plugged into the connector 1108 as shown in FIG> 11C. After installing the drape 1110 and sleeve 1112 and plugging the plug 1114 to the connector 1108, sterile adhesive tape is wrapped around the sleeve 1112 to seal the sleeve 1112. In some implementations, the sleeve 1112 is integrated with (e.g., part of) the sterile drape 1110. In some implementations, the sleeve 1112 is separate from the drape 1110 and the interface between the sleeve 1112 and the sterile drape 1110 is sealed using sterile adhesive tape 1116 or another sterile adhesive material.

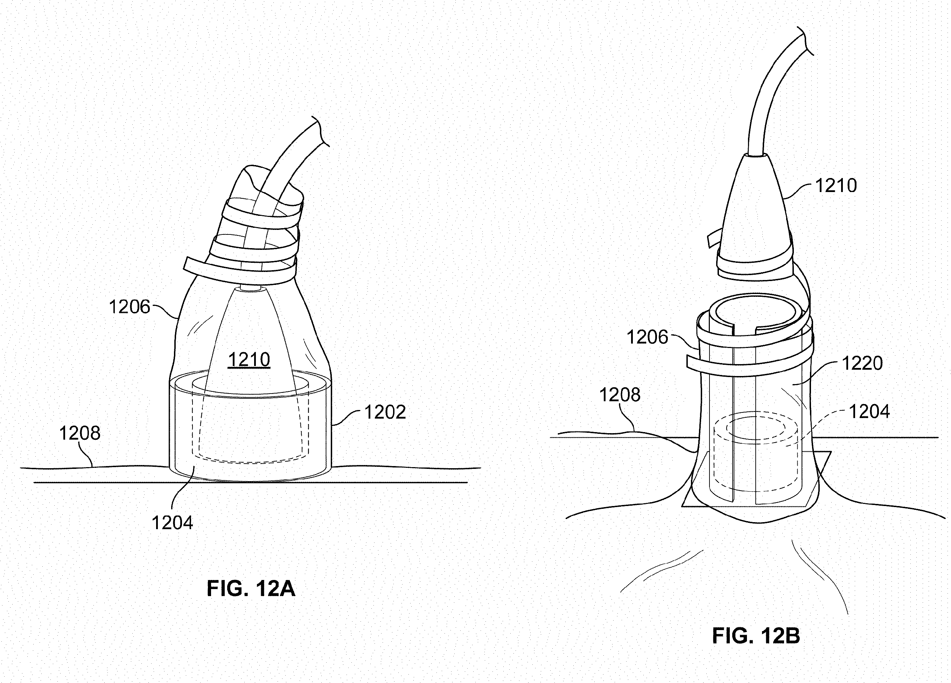

FIGS. 12A-D are illustrations of an example stiffeners for providing a stiffer sleeve 1206 on a sterile drape 1208. The stiffener may be used to simplify passing the plug 1210 though the flexible sleeve 1206. The stiffener may be rigid (e.g., made from plastic) or semi-rigid member (e.g. made from rubber, adhesive tape, tape reinforcement). As shown in FIG. 12A, the sterile drape stiffener 1202 may be takes integrated into the drape 1208. The stiffener the form of a supporting tube 1220 as illustrated in FIG. 12B. The supporting tube 1220 may be removable. The supporting tube 1220 may have a longitudinal opening which allows the plug 1210 to be plugged into the connector 1204 through the supporting tube 1220. The supporting tube 1220 may be inserted before or after applying the drape 1208 to the robot. In some implementations, the supporting tube 1220 may be removably attached to the drape. In some implementations, after inserting the plug 1210 into the connector 1204, the supporting tube 1220 may be removed and sterile adhesive tape is applied to the sleeve 1206 (e.g., as described above).

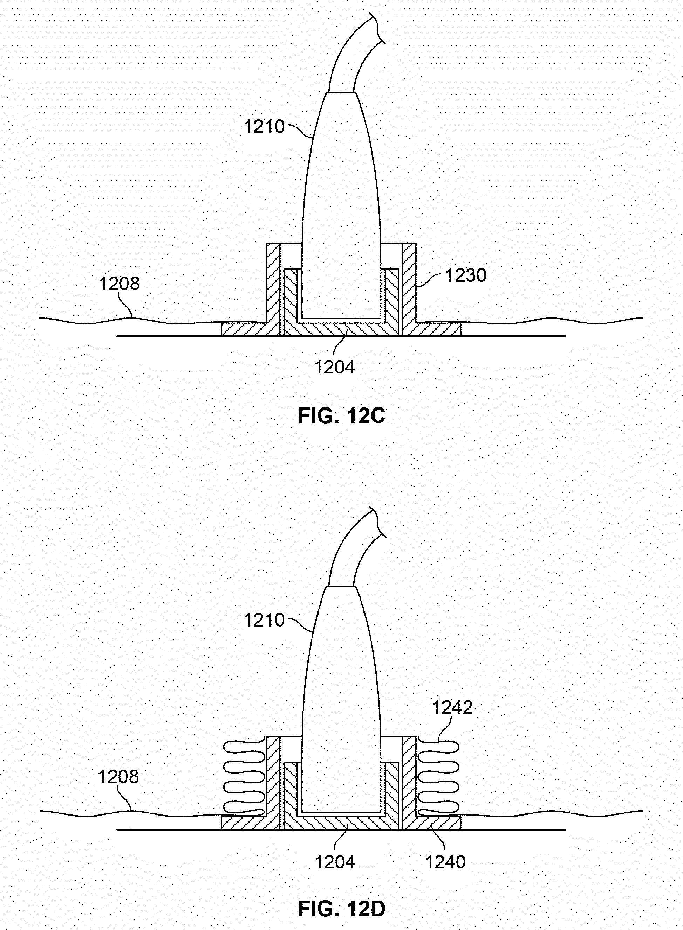

In some implementations, the connector adapter 1230 is integrated into the drape 1208 as shown in FIG. 12C. The connector adapter 1230 may fit around the connector 1204 and allow for simple access to the connector 1204 by the plug 1210. Sterile adhesive tape may be applied to the sleeve 1206 after the plug 1210 is inserted into the connector 1204 (e.g., as described above).

In some implementations, a drape 1208 with a folded section 1242 is placed around the connector 1240 as shown in FIG. 12D. After the plug 1210 is inserted into the connector 1240, the folded section 1242 of the drape 1208 is unfolded and sterile adhesive tape is applied (e.g., as described above).

FIG. 13 is an illustration of an example system for passing electrical signals through a sterile drape. FIG. 13 illustrates a sterile adapter 1306 (e.g., as described above) and a connector opening 1308. In some implementations, a cable and plug are attached to the drape 1302 before draping the robot. For example, a scrub nurse may do this on a sterile table. The cable may be passed through the sleeve 1304. In some implementations, the drape 1302 and/or sleeve 1304 is/are folded to facilitate passing the cable through the sleeve 1304. After passing the cable through the sleeve 1304, it may be sealed using sterile adhesive tape (e.g., as described above). After sealing the plug and sleeve 1304, the drape 1302 may be applied to the robot and the plug may be connected to the connector by manipulating it from the sterile side of the drape 1302.

In view of the structure, functions and apparatus of the systems and exemplary methods described here, in some implementations, a system and exemplary method for performing surgery with a robotic surgical system are provided. Having described certain exemplary implementations of methods and apparatus for supporting a robotic surgical system, it will now become apparent to one of skill in the art that other implementations incorporating the concepts of the disclosure may be used. Therefore, the invention should not be limited to certain implementations, but rather should be limited only by the scope of the following claims.

Throughout the description, where apparatus and systems are described as having, including, or comprising specific components, or where processes and methods are described as having, including, or comprising specific steps, it is contemplated that, additionally, there are apparatus, and systems of the disclosed technology that consist essentially of, or consist of, the recited components, and that there are processes and methods according to the disclosed technology that consist essentially of, or consist of, the recited processing steps.

It should be understood that the order of steps or order for performing certain action is immaterial so long as the disclosed technology remains operable. Moreover, two or more steps or actions may be conducted simultaneously.

## Claims

1. A sterile drape (1208) for covering a surgical robotic arm and preventing contamination of a sterile field, the sterile drape comprising a flexible covering with a sterile outer surface and a sterile drape sleeve (1206) configured to receive an electrical plug (1210) therethrough, said covering being at least partially conformable to a surgical robotic arm, wherein the sterile drape sleeve is configured to be sealed around a portion of the electrical plug using a sterile adhesive tape (1012), wherein the sterile drape sleeve is configured to surround at least a portion of a connector (1204) of the surgical robotic arm and extend outwardly from the connector (1204), wherein the sterile drape includes a drape stiffener in the form of a supporting tube (1220) configured to allow the plug to pass through the sterile drape sleeve.

2. The sterile drape (1208) of any one of the preceding claims, wherein the connector (1204) is configured to permit electrical contact between the surgical robotic arm (e.g., an actuator of the robotic arm) and a sterile manipulator (e.g., sterile handle) of the robotic arm when the sterile manipulator is separated from the surgical robotic arm by the flexible covering.

3. The sterile drape (1208) of any one of the preceding claims, wherein the sterile drape is configured to be attached to the sterile drape before draping the robotic surgical arm.

4. The sterile drape (1208) of any one of the preceding claims, wherein the connector (1204) is configured to receive the electrical plug (1210) after the electrical plug is passed through the sterile drape sleeve.

5. The sterile drape (1208) of any one of the preceding claims, wherein the connector (1204) is configured to receive the electrical plug (1210) after (i) the electrical plug is passed through the sterile drape sleeve and (ii) the sterile drape sleeve is sealed around a portion of the electrical plug using a sterile adhesive tape (1012).

6. The sterile drape (1208) of any one of the preceding claims, wherein the sterile drape is configured to be attached to the sterile drape after draping the robotic surgical arm.

7. The sterile drape (1208) of claim 1, wherein the drape stiffener (1220) is made of a semi-rigid material (e.g., rubber, adhesive tape, tape reinforcement).

8. The sterile drape (1208) of claim 1, wherein the drape stiffener (1220) is made of a rigid material (e.g., plastic).

9. The sterile drape (1208) of any one of claims 7 or 8, wherein the drape stiffener (1220) is integrated in the sterile drape.

10. The sterile drape (1208) of any one of claims 7 to 9, wherein the drape stiffener (1220) is removable.

11. The sterile drape (1208) of any one of claims 7 to 10, wherein the drape stiffener (1220) is configured to be inserted before the sterile drape is applied to the robot surgical arm.

12. The sterile drape (1208) of any one of claims 7 to 10, wherein the drape stiffener (1220) is configured to be inserted after the sterile drape is applied to the robot surgical arm.

13. The sterile drape (1208) of any one of claims 7 to 12, wherein the drape stiffener (1220) is configured to be removed after the sterile drape is applied to the robot surgical arm.

14. The sterile drape (1208) of any one of claims 7 to 13, wherein the drape stiffener (1220) is configured to be removed after (i) the sterile drape is applied to the robot surgical arm and (ii) the sterile drape sleeve is sealed around a portion of the electrical plug (1210) using a sterile adhesive tape (1012).

## Patentansprüche

1. Steriles Abdecktuch (1208) zum Überziehen eines chirurgischen Roboterarms und zum Verhindern einer Kontamination eines sterilen Bereichs, wobei das sterile Abdecktuch einen flexible Überzug mit einer sterilen Außenoberfläche und eine sterile Abdecktuchhülle (1206) umfasst, die konfiguriert ist, um einen elektrischen Stecker (1210) dahindurch aufzunehmen, wobei der Überzug wenigstens teilweise an einen chirurgischen Roboterarm anpassbar ist, wobei die sterile Abdecktuchhülle konfiguriert ist, um unter Verwendung eines sterilen Klebebands (1012) um einen Abschnitt des elektrischen Steckers herum abgedichtet zu werden, wobei die sterile Abdecktuchhülle konfiguriert ist, um wenigstens einen Abschnitt eines Verbinders (1204) des chirurgischen Roboterarms zu umgeben und sich von dem Verbinder (1204) nach außen zu erstrecken, wobei das sterile Abdecktuch eine Abdecktuchversteifung in der Form eines Stützrohrs (1220) beinhaltet, das konfiguriert ist, um zu ermöglichen, dass der Stecker durch die sterile Abdecktuchhülle hindurchgeht.

2. Steriles Abdecktuch (1208) nach einem der vorhergehenden Ansprüche, wobei der Verbinder (1204) konfiguriert ist, um einen elektrischen Kontakt zwischen dem chirurgischen Roboterarm (z. B. einem Stellantrieb des Roboterarms) und einem sterilen Manipulator (z. B. einem sterilen Griff) des Roboterarms zu ermöglichen, wenn der sterile Manipulator von dem chirurgischen Roboterarm durch den flexiblen Überzug getrennt ist.

3. Steriles Abdecktuch (1208) nach einem der vorhergehenden Ansprüche, wobei das sterile Abdecktuch konfiguriert ist, um vor dem Abdecken des chirurgischen Roboterarms an dem sterilen Abdecktuch befestigt zu werden.

4. Steriles Abdecktuch (1208) nach einem der vorhergehenden Ansprüche, wobei der Verbinder (1204) konfiguriert ist, um den elektrischen Stecker (1210) aufzunehmen, nachdem der elektrische Stecker durch die sterile Abdecktuchhülle hindurchgeführt wurde.

5. Steriles Abdecktuch (1208) nach einem der vorhergehenden Ansprüche, wobei der Verbinder (1204) konfiguriert ist, um den elektrischen Stecker (1210) aufzunehmen, nachdem (i) der elektrische Stecker durch die sterile Abdecktuchhülle hindurchgeführt wurde und (ii) die sterile Abdecktuchhülle um einen Abschnitt des elektrischen Steckers unter Verwendung eines sterilen Klebebandes (1012) abgedichtet wurde.

6. Steriles Abdecktuch (1208) nach einem der vorhergehenden Ansprüche, wobei das sterile Abdecktuch konfiguriert ist, um nach dem Abdecken des chirurgischen Roboterarms an dem sterilen Abdecktuch befestigt zu werden.

7. Steriles Abdecktuch (1208) nach Anspruch 1, wobei die Abdecktuchversteifung (1220) aus einem halbstarren Material (z. B. Kautschuk, Klebeband, Bandverstärkung) hergestellt ist.

8. Steriles Abdecktuch (1208) nach Anspruch 1, wobei die Abdecktuchversteifung (1220) aus einem starren Material (z. B. Kunststoff) hergestellt ist.

9. Steriles Abdecktuch (1208) nach einem der Ansprüche 7 oder 8, wobei die Abdecktuchversteifung (1220) in das sterile Abdecktuch integriert ist.

10. Steriles Abdecktuch (1208) nach einem der Ansprüche 7 bis 9, wobei die Abdecktuchversteifung (1220) abnehmbar ist.

11. Steriles Abdecktuch (1208) nach einem der Ansprüche 7 bis 10, wobei die Abdecktuchversteifung (1220) konfiguriert ist, um eingesetzt zu werden, bevor das sterile Abdecktuch an dem chirurgischen Roboterarm angebracht wird.

12. Steriles Abdecktuch (1208) nach einem der Ansprüche 7 bis 10, wobei die Abdecktuchversteifung (1220) konfiguriert ist, um eingesetzt zu werden, nachdem das sterile Abdecktuch an dem chirurgischen Roboterarm angebracht wurde.

13. Steriles Abdecktuch (1208) nach einem der Ansprüche 7 bis 12, wobei die Abdecktuchversteifung (1220) konfiguriert ist, um abgenommen zu werden, nachdem das sterile Abdecktuch an dem chirurgischen Roboterarm angebracht wurde.

14. Steriles Abdecktuch (1208) nach einem der Ansprüche 7 bis 13, wobei die Abdecktuchversteifung (1220) konfiguriert ist, um abgenommen zu werden, nachdem (i) das sterile Abdecktuch an dem chirurgischen Roboterarm angebracht wurde und (ii) die sterile Abdecktuchhülle um einen Abschnitt des elektrischen Steckers (1210) unter Verwendung eines sterilen Klebebandes (1012) abgedichtet wurde.

## Revendications

1. Drap stérile (1208) destiné à recouvrir un bras robotique chirurgical et empêcher la contamination d'un champ stérile, le drap stérile comprenant un revêtement flexible avec une surface extérieure stérile et un manchon de drap stérile (1206) conçu pour recevoir une fiche électrique (1210) à travers celui-ci, ledit revêtement étant au moins partiellement conformable à un bras robotique chirurgical, dans lequel le manchon de drap stérile est conçu pour être scellé autour d'une partie de la fiche électrique à l'aide d'un ruban adhésif stérile (1012), dans lequel le manchon de drap stérile est conçu pour entourer au moins une partie d'un connecteur (1204) du bras robotique chirurgical et s'étend vers l'extérieur depuis le connecteur (1204), dans lequel le drap stérile comporte un raidisseur de drap sous la forme d'un tube de support (1220) conçu pour permettre au bouchon de passer à travers le manchon de drap stérile.

2. Drap stérile (1208) selon l'une quelconque des revendications précédentes, dans lequel le connecteur (1204) est conçu pour permettre un contact électrique entre le bras robotique chirurgical (par exemple, un actionneur du bras robotique) et un manipulateur stérile (par exemple, une poignée stérile) du bras robotique lorsque le manipulateur stérile est séparé du bras robotique chirurgical par le revêtement souple.

3. Drap stérile (1208) selon l'une quelconque des revendications précédentes, dans lequel le drap stérile est conçu pour être fixé au champ stérile avant le drapage du bras robotique chirurgical.

4. Drap stérile (1208) selon l'une quelconque des revendications précédentes, dans lequel le connecteur (1204) est conçu pour recevoir la fiche électrique (1210) après le passage de la fiche électrique à travers le manchon de drap stérile.

5. Drap stérile (1208) selon l'une quelconque des revendications précédentes, dans lequel le connecteur (1204) est conçu pour recevoir la fiche électrique (1210) après (i) le passage de la fiche électrique à travers le manchon de drap stérile et (ii) le manchon de drap stérile est scellé autour d'une partie de la fiche électrique à l'aide d'un ruban adhésif stérile (1012).

6. Drap stérile (1208) selon l'une quelconque des revendications précédentes, dans lequel le drap stérile est conçu pour être fixé au drap stérile après le drapage du bras robotique chirurgical.

7. Drap stérile (1208) selon la revendication 1, dans lequel le raidisseur de drap (1220) est constitué d'un matériau semi-rigide (par exemple, caoutchouc, ruban adhésif, renfort de ruban).

8. Drap stérile (1208) selon la revendication 1, dans lequel le raidisseur de drap (1220) est constitué d'un matériau rigide (par exemple en plastique).

9. Drap stérile (1208) selon l'une quelconque des revendications 7 ou 8, dans lequel le raidisseur de drap (1220) est intégré dans le drap stérile.

10. Drap stérile (1208) selon l'une quelconque des revendications 7 à 9, dans lequel le raidisseur de drap (1220) est amovible.

11. Drap stérile (1208) selon l'une quelconque des revendications 7 à 10, dans lequel le raidisseur de drap (1220) est conçu pour être inséré avant l'application du drap stérile sur le bras robotique chirurgical.

12. Drap stérile (1208) selon l'une quelconque des revendications 7 à 10, dans lequel le raidisseur de drap (1220) est conçu pour être inséré après l'application du drap stérile sur le bras robotique chirurgical.

13. Drap stérile (1208) selon l'une quelconque des revendications 7 à 12, dans lequel le raidisseur de drap (1220) est conçu pour être retiré après l'application du drap stérile sur le bras robotique chirurgical.

14. Drap stérile (1208) selon l'une quelconque des revendications 7 à 13, dans lequel le raidisseur de champ (1220) est conçu pour être retiré après (i) que l'application du drap stérile sur le bras robotique chirurgical et (ii) le manchon de drap stérile est scellé autour d'une partie de la fiche électrique (1210) à l'aide d'un ruban adhésif stérile (1012).
